(19) Europäisches Patentamt
European Patent Office
Office européen des brevets

(11) Veröffentlichungsnummer: **0 226 051**
**A1**

# (12) EUROPÄISCHE PATENTANMELDUNG

(21) Anmeldenummer: 86115784.0

(22) Anmeldetag: 13.11.86

(51) Int. Cl.⁴: A 01 N 47/36
C 07 D 333/70, C 07 D 333/7-
8

(30) Priorität: 26.11.85 DE 3541630

(43) Veröffentlichungstag der Anmeldung:
24.06.87 Patentblatt 87/26

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Hallenbach, Werner, Dr.
Kleiststrasse 10
D-4018 Langenfeld(DE)

(72) Erfinder: Lindel, Hans, Dr.
Carl-Duisberg-Strasse 321
D-5090 Leverkusen 1(DE)

(72) Erfinder: Brandes, Wilhelm, Dr.
Eichendorffstrasse 3
D-5653 Leichlingen(DE)

## (54) Fungizide Verwendung von Tetrahydrobenzothienylharnstoff-Derivaten.

(57) Fungizide Verwendung von Tetrahydrobenzothienylharnstoff-Derivaten der Formel (I)

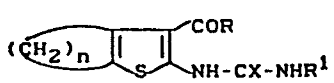

(I)

in welcher

X, R, $R^1$ und n die in der Beschreibung gegebenen Bedeutungen haben.

EP 0 226 051 A1

BAYER AKTIENGESELLSCHAFT
Konzernverwaltung RP
Patentabteilung

5090 Leverkusen, Bayerwerk
Bas/ABc 25. NOV. 1985
IIb

# Fungizide Verwendung von Tetrahydrobenzothienylharnstoff-Derivaten

Die vorliegende Erfindung betrifft die Verwendung von Tetrahydrobenzothienylharnstoff-Derivaten als Fungizide im Pflanzenschutz.

Es ist bereits bekannt geworden, daß bestimmte Thienylharnstoffe, wie z.B. 1-(4,5-Dimethyl-3-ethoxycarbonyl-2-thienyl)-3-methylharnstoff, eine gute fungizide Wirksamkeit besitzen (vgl. z.B. US-PS 3 823 161).

Außerdem sind 4,5-Tetramethyl-thienyl-harnstoff-Derivate, wie z.B. das 2-Phenylureido-3-carbethoxy-4,5-tetramethylen-thiophen, als Mittel z.B. zur Förderung der Reifung von Zuckerrohr bekannt (vgl. DE-OS 2 627 935). Die Verwendung als Fungizid ist nicht bekannt.

Es wurde gefunden, daß speziell die Verbindungen der Formel (I)

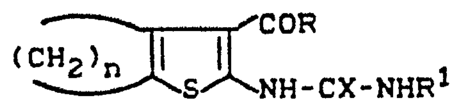

(I)

in welcher

n für die Zahlen 3, 4, 5 oder 6 steht,

X für Sauerstoff oder Schwefel steht,

R für $C_1$-$C_4$-Alkoxy oder Hydroxy steht und

$R^1$ für $C_1$-$C_4$-Alkyl steht,

als Fungizide im Pflanzenschutz verwendet werden können.

Überraschenderweise zeigen die Tetrahydrobenzothienylharnstoff-Derivate der Formel (I) eine erheblich höhere fungizide Wirkung als die aus dem Stand der Technik bekannte Verbindung 1-(4,5-Dimethyl-3-ethoxy-carbonyl-2-thienyl)-3-methylharnstoff (vgl. z.B. US-PS 3 823 161).

Der $C_1$-$C_4$-Alkoxyrest R bedeutet geradkettiges oder verzweigtes Alkoxy mit 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Der $C_1$-$C_4$-Alkylrest $R^1$ bedeutet geradkettiges oder verzweigtes Alkyl mit 1 bis 4 Kohlenstoffatomen. Beispielhaft seien Methyl, Ethyl, n-Propyl, i-Propyl, n-Butyl, i-Butyl, sec.-Butyl oder tert.-Butyl genannt.

X steht vorzugsweise für Sauerstoff.

Die erfindungsgemäß zu verwendenden Tetrahydrobenzothienylharnstoff-Derivate sind durch die Formel (I) allgemein definiert.

In dieser Formel stehen vorzugsweise

n für die Zahlen 3, 4, 5 oder 6,

X für Sauerstoff,

R für Hydroxy, Methoxy, Ethoxy, n-Propoxy, i-Propoxy, n-Butoxy, i-Butoxy, sec.-Butoxy oder tert.-Butoxy und

$R^1$ für Methyl, Ethyl, n-Propyl, i-Propyl, n-Butyl, i-Butyl, sec.-Butyl oder tert.-Butyl.

Besonders bevorzugt werden diejenigen Verbindungen der Formel (I) verwendet, in denen

n für die Zahlen 3, 4 oder 5 steht,

X für Sauerstoff steht,

R für Hydroxy, Methoxy, Ethoxy, n-Propoxy oder tert.-Butoxy steht und,

$R^1$ für Methyl, Ethyl, i-Propyl oder n-Butyl steht.

Die erfindungsgemäß zu verwendenden Wirkstoffe der Formel (I) sind teilweise bekannt und/oder lassen sich in einfacher Weise nach bekannten Methoden herstellen (vgl. z.B. EP-OS 4 931, DE-AS 20 40 579, DE-AS 21 22 636 (= US-PS 3 823 161), DE-AS 26 27 935 und eine eigene ältere nicht vorveröffentlichte Anmeldung P 35 29 247.4 vom 16.08.1985) So lassen sich z.B. 2-Amino-tetrahydrobenzothiophen-Derivate mit Isocyanaten oder Isothiocyanaten gemäß dem folgenden Formelschema umsetzen:

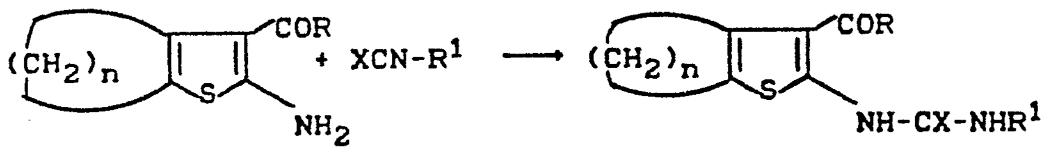

Die Reaktion wird üblicherweise bei Normaldruck und einer Temperatur von 20°C bis 70°C durchgeführt, gegebenenfalls in Anwesenheit einer Hilfsbase und in Gegenwart inerter Verdünnungsmittel, wie z.B. Toluol, Chloroform und Pyridin.

Die erfindungsgemäß zu verwendenden Wirkstoffe weisen eine starke fungizide Wirkung auf und können zur Bekämpfung von unerwünschten Pilzen praktisch eingesetzt werden. Die Wirkstoffe sind für den Gebrauch als Fungizide im Pflanzenschutz geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;

Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus

organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß zu verwendenden Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die erfindungsgemäß zu verwendenden Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik (A) zeigt in diesem Test z. B. die Verbindung gemäß dem folgenden Beispiel:

Tabelle A

Botrytis-Test (Bohne)/protektiv

| Wirkstoff | Befall in % bei einer Wirkstoffkonzentration von 100 ppm |
| --- | --- |
| 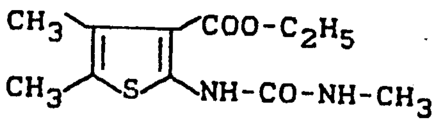 (bekannt) (A) | 29 |
| 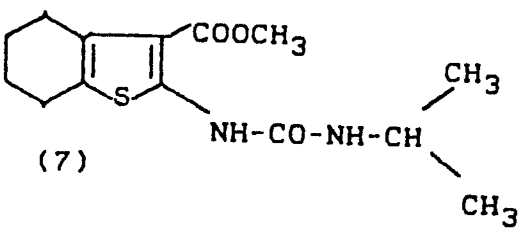 (7) | 17 |
| 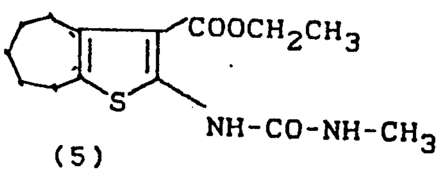 (5) | 11 |
| 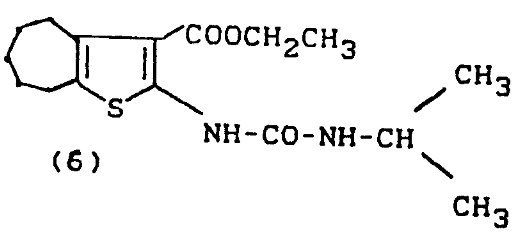 (6) | 17 |

0226051

## Beispiel B

Botrytis-Test (Reben)/protektiv

Lösungsmittel: 4,7 Gewichtsteile Aceton
Emulgator: 0,8 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern ausgewertet.

Eine deutliche Überlegenheit in Wirksamkeit gegenüber dem Stand der Technik (A) zeigt in diesem Test z.B. die Verbindung gemäß dem folgenden Beispiel:

## Tabelle B

Botrytis-Test (Rebe)/protektiv

| Wirkstoff | Befall in % bei einer Wirkstoffkonzentration von 25 ppm |
|---|---|
| (bekannt) (A) 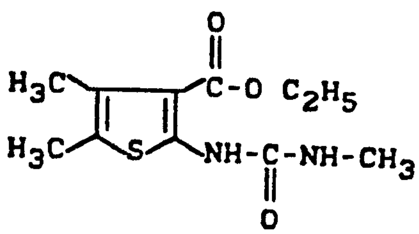 | 32 |
| 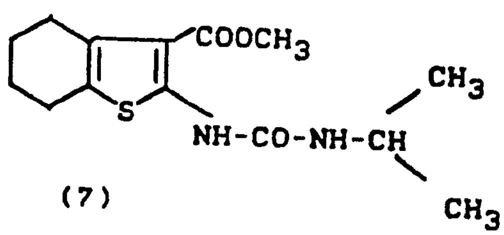 (7) | 6 |

## Herstellungsbeispiele

### Beispiel 1

Herstellung von

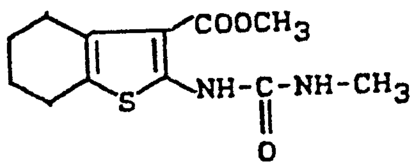

4,5 g (0,021 mol) 2-Amino-3-methoxycarbonyl-tetrahydrobenzothiophen und 1,4 g (0,024 mol) Methylisocyanat werden in 100 ml trockenem Chlorform 24 h unter Rückfluß erhitzt. Dann wird die Choroformphase dreimal mit je 50 ml Wasser gewaschen, über Natriumsulfat getrocknet und eingedampft. Das anfallende Rohprodukt wird aus Ethanol umkristallisiert.
Ausbeute: 3,2 g (58 % der Theorie), Schmelzpunkt 167° C (Zers.)

Analog Beispiel 1 können folgende Verbindungen der Formel (I)

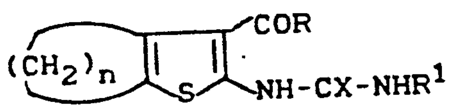
(I)

hergestellt werden:

| Bsp.-Nr. | n | R | $R^1$ | X | Schmelzpunkt |
|---|---|---|---|---|---|
| 2 | 3 | $-OC_2H_5$ | $CH_3$ | O | 165 |
| 3 | 3 | $-OC_2H_5$ | $i-C_3H_7$ | O | 145 |
| 4 | 4 | $-OC_4H_9-t$ | $CH_3$ | O | 150 |
| 5 | 5 | $-OC_2H_5$ | $CH_3$ | O | 148 |
| 6 | 5 | $-OC_2H_5$ | $i-C_3H_7$ | O | 113 |
| 7 | 4 | $-OCH_3$ | $i-C_3H_7$ | O | 165 |
| 8 | 4 | $-OCH_3$ | $n-C_4H_9$ | O | 130 |
| 9 | 4 | $-OH$ | $i-C_3H_7$ | O | 174 |

Patentansprüche

1. Fungizide Verwendung im Pflanzenschutz von Tetrahydrobenzothienylharnstoff-Derivaten der Formel (I)

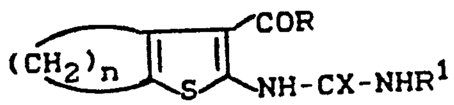

(I)

in welcher

n für die Zahlen 3, 4, 5 oder 6 steht,
X für Sauerstoff oder Schwefel steht,
R für $C_{1-4}$-Alkoxy oder Hydroxy steht und
$R^1$ für $C_{1-4}$-Alkyl steht.

2. Fungizide Verwendung im Pflanzenschutz von Tetrahydrobenzothienylharnstoff-Derivaten der Formel (I) gemäß Anspruch 1, worin

n für die Zahlen 3, 4, 5 oder 6 steht,
X für Sauerstoff steht,
R für Methoxy, Ethoxy, n-Propoxy, i-Propoxy, n-Butoxy, i-Butoxy, sec.-Butoxy, tert.-Butoxy oder Hydroxy steht und

$R^1$ für Methyl, Ethyl, n-Propyl, i-Propyl, n-Butyl, i-Butyl, sec.-Butyl oder tert.-Butyl steht.

3. Fungizide Verwendung im Pflanzenschutz von Tetrahydrobenzothienylharnstoff-Derivaten der Formel (I) gemäß Anspruch 1, worin

n für die Zahlen 3, 4 oder 5 steht,

X für Sauerstoff steht,

R für Hydroxy, Methoxy, Ethoxy, n-Propoxy oder tert.-Butoxy steht und

$R^1$ für Methyl, Ethyl, i-Propyl oder n-Butyl steht.

4. Fungizide Mittel für den Pflanzenschutz, gekennzeichnet durch einen Gehalt an Tetrahydrobenzothienylharnstoff-Derivaten der Formel (I) gemäß den Ansprüchen 1 bis 3.

5. Verfahren zur Herstellung von fungiziden Mitteln für den Pflanzenschutz, dadurch gekennzeichnet, daß man Tetrahydrobenzothienylharnstoff-Derivate der Formel (I) gemäß den Ansprüchen 1 bis 3 mit Streck- und/oder Verdünnungsmitteln vermischt.

Europäisches Patentamt

**EUROPÄISCHER RECHERCHENBERICHT**

0226051

Nummer der Anmeldung

EP 86 11 5784

Seite 2

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | CHEMICAL ABSTRACTS, Band 92, Nr. 9, 3. März 1980, Seite 650, Zusammenfassung Nr. 76205r, Columbus, Ohio, US; V.J. RAM: "Thiophenes, pyrazolothiophenes and pyrimidothiophenes as pesticides", & ARCH. PHARM. (Weinheim, Ger.) 1979, 312(9), 726-33<br>* Insgesamt *<br>----- | 1-5 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 11-03-1987 | FLETCHER A.S. |

KATEGORIE DER GENANNTEN DOKUMENTE

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0226051

Nummer der Anmeldung

EP 86 11 5784

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | JOURNAL OF PHARMACEUTICAL SCIENCES, Band 65, Nr. 5, Mai 1976, Seiten 660-664, Washington, D.C., US; M.B. DEVANI et al.: "Synthesis of 3-substituted thieno[2,3-d]pyrimidin-4(3H)-one-2-mercaptoacetic acids and their ethyl esters for pharmacological screening" * Insgesamt * | 1,4,5 | A 01 N 47/36<br>C 07 D 333/70<br>C 07 D 333/78 |
| | --- | | |
| A | Idem | 2,3 | |
| | --- | | |
| D,A | DE-A-2 122 636 (ESSO RESEARCH AND ENGINEERING) * Seite 3, Verbindungen 10,22; Patentansprüche 1,26-28 * | 1-5 | |
| | --- | | **RECHERCHIERTE SACHGEBIETE (Int Cl 4)** |
| D,A | DE-A-2 627 935 (W.R. GRACE & CO.) * Patentansprüche 1,7-12,14,24-27 * | 1-5 | A 01 N |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 11-03-1987 | FLETCHER A.S. |

KATEGORIE DER GENANNTEN DOKUMENTE

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82